# EUROPÄISCHE PATENTSCHRIFT

(11) **EP 0 436 801 B1**
(45) Veröffentlichungstag und Bekanntmachung des Hinweises auf die Patenterteilung: **22.09.1993**
(21) Anmeldenummer: 90121757.0
(22) Anmeldetag: 14.11.1990
(51) Int. Cl.: C07C 31/30, C07C 29/68, C08F 4/64

(54) **Verfahren zur Herstellung sphärischer Partikel von Magnesiumalkoxid**
Preparation of spherical magnesium alkoxide particles
Procédé pour la fabrication de particules sphériques d'alcoxyde de magnésium

(30) Priorität: 12.01.1990 DE 4000697
(43) Veröffentlichungstag der Anmeldung: 17.07.1991
(73) Patentinhaber: HÜLS AKTIENGESELLSCHAFT, 45764 Marl (DE)
(72) Erfinder: Horns, Udo, Dr., Theodore, AL 36590 (US); Jostmann, Thomas, Dr., W-4358 Haltern (DE); Kassmann, Klaus-Dieter, Dr., W-4370 Marl (DE); Matthes, Reinhard, Dr., W-7888 Rheinfelden (DE); Rauleder, Hartwig, Dr., W-7888 Rheinfelden (DE)

(56) Entgegenhaltungen:
- EP-A- 0 216 402
- EP-A- 0 236 082
- DE-B- 1 806 549

## Beschreibung

Diese Erfindung bezieht sich auf feinteiliges, sphärisches Magnesiumalkoxid, ein Verfahren zu dessen Herstellung sowie dessen Verwendung zur Herstellung von hochaktiven Katalysatoren für die Polymerisation von α-Olefinen.

Katalysatoren für die Olefinpolymerisation können nach einer Vielzahl von Verfahren durch Reaktion einer festen Komponente, die in der Regel Magnesium, Titan und Halogen, vorzugsweise Chlor, enthält, mit einer aluminiumorganischen Verbindung hergestellt werden. Die Aktivität und Stereospezifität derartiger Trägerkatalysatoren wird üblicherweise durch Einbau eines Elektronendonors (Lewisbase) in die Trägerkomponente und durch Komplexierung der aluminiumorganischen Verbindung mit einem weiteren Elektronendonor verbessert. Es ist bekannt, hierbei Magnesiumalkoxide als Ausgangsstoffe für die Herstellung derartiger Trägerkatalysatoren einzusetzen. Vorzugsweise werden in diesem Fall die Magnesiumalkoxid-Partikel mit Hilfe eines geeigneten Halogenierungsmittels wie Benzoylchlorid, Thionylchlorid oder Titantetrachlorid in Gegenwart eines Elektronendonors wie z. B. Ethylbenzoat halogeniert. Wenn zunächst andere Halogenierungsmittel als Titantetrachlorid verwendet werden, muß durch anschließende Umsetzung mit Titantetrachlorid dieses in der benötigten Menge in die feste Komponente eingebaut werden, wobei üblicherweise ein Gehalt von 2 bis 4,5 Gew.-% Titan benötigt wird. Bezüglich der Einzelheiten der Katalysatorpräparation sei auf die EP-OSS 0 216 402 und 0 236 082 sowie die dort genannte Literatur verwiesen.

Da bei der Polymerisation von α-Olefinen die Morphologie der entstehenden Polymerpartikel eine getreue Wiedergabe der Morphologie des Katalysators und diese wiederum eine exakte Abbildung derjenigen des eingesetzten Magnesiumalkoxids ist (siehe z. B. J. Rudolph und J. Gross, Die Angewandte Makromolekulare Chemie 36 (1974) 195 - 197), muß man zur Erzielung definierter Eigenschaften des gewünschten Polymeren auch ein Magnesiumalkoxid von definierter Morphologie verwenden.

In der EP-OS 0 236 082 wird die Herstellung sphärischer bis rosinenförmiger Partikel von Magnesiumalkoxiden durch konventionelles Sprühtrocknen beschrieben. Hierbei wird eine vorzugsweise alkoholische Lösung eines carboxylierten Magnesiumalkoxids durch eine nicht näher bezeichnete Düse oder über eine rotierende Tellerscheibe in ein heißes Begleitgas versprüht; dieses Begleitgas kann im Gleich- oder im Gegenstrom geführt werden. Obwohl bei Temperaturen von 40 bis 120 °C gearbeitet werden kann, wird für ethanolische Lösungen der Bereich von 50 bis 90 °C bevorzugt, da nur hier die gewünschten sphärischen bis rosinenförmigen Partikel erzeugt werden können. Wie aus den Beispielen hervorgeht, werden bei 100 - 120 °C dagegen Hohlkugeln erhalten, von denen viele in nußschalenähnliche Fragmente zersprungen sind.

Praktisch alle der nach dem Verfahren der EP-OS 0 236 082 erhaltenen Partikel haben einen Durchmesser im Bereich von 2 bis 250 µm; vorzugsweise liegen 90 Gew.-% der Teilchen im Bereich von 10 bis 40 µm mit einem Gewichtsmittel bei etwa 20 µm.

Nach dem Sprühtrocknen wird das gebundene CO₂ durch mehrtägiges Erhitzen im Stickstoffstrom bei Temperaturen von anfangs 70 °C bis schließlich 150 °C wieder ausgetrieben. Als Alternative zu diesem aufwendigen Prozeß soll das erhaltene carboxylierte Magnesiumalkoxid direkt weiter umgesetzt werden können. Der erhaltene Katalysator soll sowohl für die Gasphasen- als auch für die Flüssigphasenpolymerisation verwendet werden können.

Das in der EP-OS 0 236 082 beschriebene Verfahren zur Herstellung von sphärischen Partikeln von Magnesiumalkoxid kann aus mehreren Gründen nicht befriedigen:
- Die Herstellung von feinteiligerem Magnesiumalkoxid mit einem mittleren Durchmesser von unter 10 µm, wie es speziell für die Herstellung von Katalysatoren für die Flüssigphasenpolymerisation wünschenswert wäre, ist auf diesem Wege nicht möglich.
- Die relativ niedrige Trocknungstemperatur beim Sprühtrocknen führt zu Produkten, die eine beträchtliche Restfeuchte mit der Gefahr von Verklumpungen und Wandanbackungen besitzen.
- Zur Entfernung des gebundenen CO₂ wird eine umständliche Nachbehandlung benötigt. Man kann alternativ auf diese Nachbehandlung verzichten, erhält auf diese Weise jedoch nur einen wesentlich weniger aktiven Katalysator.

In der EP-OS 0 216 402 wird ein mehrstufiges Verfahren beschrieben, sphärisches, gemischtes Magnesiumalkoxid der Formel Mg(OR)₂₋ₐ(OR')ₐ herzustellen (a = 0 bis 0,5). Im bevorzugten Falle (R = C₂H₅; R' = CH₃) geht man folgendermaßen vor:
1) Aus Magnesium und Ethanol wird eine ethanolische Aufschlämmung von Magnesiumethoxid hergestellt; das Produkt wird isoliert und getrocknet.
2) Das trockene Magnesiumethoxid wird in Methanol gelöst; die Lösung wird bei 15 bis 200 °C, bevorzugt bei 30 bis 70 °C, sprühgetrocknet, wobei sphärische Partikel mit einem Durchmesser von 5 bis 30 µm, bevorzugt 10 bis 18 µm entstehen. An dieser Stelle liegt ein gemischtes Alkoxid mit einem überwiegenden Anteil an Methoxidgruppen vor; dies ist in dieser Form zur Herstellung von Olefinpolymerisationskatalysatoren ungeeignet.
3) Das Produkt aus 2) wird in Ethanol aufgeschlämmt und das im Gleichgewicht gebildete Methanol abdestilliert.
4) Das methoxidgruppenarme Produkt wird isoliert und getrocknet.

Abgesehen von der Vielzahl der Verfahrensstufen wie z. B. zweifachem Lösungsmittelwechsel und dreimaligem Trocknen wird die geringe Löslichkeit des Magnesiumethoxids in Methanol (siehe EP-OS 0 216 402, Beispiel 1) und die daraus folgende Notwendigkeit, große Lösungsmittelmengen mit entsprechendem Energieaufwand zu verdampfen, als gravierender Nachteil empfunden. Zudem enthält das Produkt immer noch einen Restgehalt an unerwünschten Methoxidgruppen.

Das primäre Ziel der vorliegenden Erfindung ist die Bereitstellung von feinteiligem, sphärischem Magnesiumalkoxid für die Herstellung eines Katalysators zur Polymerisation von α-Olefinen, der es gestattet, Polyolefine, insbesondere Polypropylen, mit hoher Stereospezifität, hoher Schüttdichte und guter Fluidisierbarkeit bei pneumatischer Förderung zu produzieren. Das Polymere soll auch ohne Extraktion einen möglichst niedrigen Aschegehalt aufweisen und darf bei der Verarbeitung keine Stippen enthalten.

Wegen der bekannten Abhängigkeit der Polyolefin-Morphologie von derjenigen des Polymerisationskatalysators und damit von der Morphologie des eingesetzten Magnesiumalkoxids ergab sich die Forderung nach einem Verfahren, das die Herstellung sphärischer, kompakter Teilchen von Magnesiumalkoxid mit einer relativ engen Teilchengrößenverteilung und einem Gewichtsmittel des Teilchendurchmessers von weniger als 10 µm gestattet. Insbesondere dürfen, um Stippenfreiheit und hohe Fluidisierbarkeit zu gewährleisten, keine nennenswerten Anteile mit Durchmessern über ca. 100 µm enthalten sein. Dies setzt Vorkehrungen voraus, um einmal eine wirksame Zerstäubung sicherzustellen und zum anderen eine Agglomeration noch feuchter Partikel im Gasraum oder an der Wandung zu verhindern.

Derart hergestelltes, bisher nicht erhältliches feinteiliges, methoxidgruppenfreies Magnesiumalkoxid läßt sich besonders vorteilhaft zur Herstellung von Katalysatoren für die Flüssigphasenpolymerisation einsetzen.

Neben der Flüssigphasenpolymerisation von Olefinen haben sich weltweit Verfahren zur Gasphasenpolymerisation etabliert, in denen die Polymerisation z. B. in einem Wirbelbett abläuft. Hierfür wird ein grobkörnigerer Katalysator benötigt; typisch sind mittlere Teilchendurchmesser von z. B. 20 µm. Es ist daher ein weiteres Ziel der vorliegenden Erfindung, ein flexibles Verfahren zur Verfügung zu stellen, bei dem in der gleichen Anlage ohne Umrüsten, lediglich durch geeignete Variation von Parametern wie z. B. Lastbereich der Düse, wahlweise das erfindungsgemäße feinteilige Magnesiumalkoxid mit einem Gewichtsmittel des Teilchendurchmessers von weniger als 10 µm oder ein grobkörnigeres Magnesiumalkoxid mit einem Gewichtsmittel von 20 µm oder mehr hergestellt werden kann. Eine derartige Flexibilität der Anlage könnte wesentlich zu deren Wirtschaftlichkeit beitragen.

Zudem sollte ein einfaches und schnelles Verfahren zur Feintrocknung und Decarboxylierung des zunächst entstandenen feinteiligen carboxylierten Magnesiumalkoxids gefunden werden.

Diese Aufgaben werden bei der vorliegenden Erfindung durch das im folgenden beschriebene Verfahren gelöst.

Das erfindungsgemäße Verfahren ist dadurch gekennzeichnet, daß eine Lösung von carboxyliertem Magnesiumalkoxid im gleichen Alkohol, der auch dem Alkoxid zugrunde liegt,
a) über eine Zweistoffdüse mit Innenzerstäubung der Art, wie sie z. B. in der DE-PS 26 27 880 beschrieben ist, und welche im Unterlastbereich mit 5 bis 30 %, bevorzugt 10 bis 25 % der Kapazität betrieben wird,
b) in ein inertes Begleitgas versprüht wird, das unter einem Druck von 1,0 bis 1,2 bar, bevorzugt 1,01 bis 1,05 bar, steht, im Gleichstrom geführt wird und auf eine relativ hohe Temperatur von 100 bis 140 °C, bevorzugt 105 bis 120 °C, vorgeheizt wurde, wobei es vorteilhaft ist, wenn der Sprühtrockner als langes, nach unten konisch zulaufendes Rohr ausgeführt ist;
c) wonach das entstandene feinteilige carboxylierte Magnesiumalkoxid getrocknet und decarboxyliert wird.

Das eingesetzte carboxylierte Magnesiumalkoxid besitzt die Formel

Mg(OR)₂ · x CO₂

mit R = C₂ bis C₄ Alkyl wie z. B. n-Propyl, n-Butyl, i-Butyl oder bevorzugt Ethyl und x = 0,2 bis 2,0. Es wird auf bekannte Weise hergestellt, indem Magnesiumspäne im entsprechenden Alkohol gelöst werden und die zunächst gebildeten Partikel von Magnesiumalkoxid durch Einleiten von trockenem CO₂ wieder in Lösung gebracht werden. Das CO₂ ist hierbei nur ein Hilfsmittel, um die Löslichkeit des Alkoxids zu erhöhen; seine Reaktion mit dem Alkoxid ist reversibel, weshalb es durch Erhitzen wieder entfernt werden kann. Je größer hierbei x ist, umso leichter wird beim Erhitzen wieder CO₂ abgegeben; um das Begleitgas, das nach Auskondensieren des Alkohols umgewälzt wird, nicht zu sehr mit CO₂ zu verunreinigen, ist es herbei ratsam, x nur so groß wie unbedingt nötig zu wählen. Im Falle von R = Ethyl ist aus diesem Grunde ein Wert für x von 1,0 bis 1,25 besonders bevorzugt.

Die Lösung kann einen Feststoffgehalt von 2 bis 40 Gew.-% besitzen. Man arbeitet üblicherweise mit Konzentrationen zwischen 20 und 40 Gew.-%, besonders bevorzugt 25 bis 30 Gew.-% Trockensubstanz.

Bei der verwendeten Düse handelt es sich um eine Zweistoffdüse mit Innenzerstäubung. Flüssigkeits- und Gasdruck müssen dabei gleich hoch sein. Es wird bei Drucken zwischen 10 und 80 bar, vorzugsweise zwischen 35 und 45 bar versprüht.

Als Treibgas und als Begleitgas kann jedes inerte Gas verwendet werden. Aus praktischen Gründen wird man trockenen und sauerstofffreien Stickstoff einsetzen. Bei Verwendung von carboxyliertem Magnesiumethoxid in Ethanol wird eine Temperatur des Begleitgases zwischen 105 und 115 °C besonders bevorzugt.

Unter diesen Bedingungen liefern Einstoffdüsen zu grobe Partikel. Auch Zerstäuberscheiben, die prinzipiell besser geeignet sind, liefern ein zu grobes Material. Mit den beschriebenen Zweistoffdüsen werden jedoch sehr gute Ergebnisse erhalten.

Der Sprühtrockner ist im allgemeinen als schlanker Turm ausgeführt, der aus einem oberen zylindrischen Teil mit einem Durchmesser- zu Längenverhältnis von 0,3 bis 0,9, vorzugsweise 0,6 bis 0,8, sowie einem anschließenden konischen Teil besteht. Es wird bevorzugt, daß dieses konische Teil länger ist als das zylindrische. Das Begleitgas wird im Gleichstrom zu- und abgeführt, wobei die entstandenen Partikel in einer geeigneten Apparatur wie einem Zyklon oder einem Filter abgeschieden werden.

Mit dieser Vorgehensweise kann man wahlweise ein sehr feinteiliges oder, falls gewünscht, auch grobkörnigeres Magnesiumalkoxid erhalten. Wegen der verwendeten Düse, die einen kleinen Sprühwinkel aufweist, sowie der Geometrie des Turms werden hierbei Wandanbackungen stark zurückgedrängt.

Für die Feintrocknung und Decarboxylierung sind relativ scharfe Bedingungen möglich, da wegen der guten Vortrocknung, verglichen mit dem aus der EP-OS 0 236 082 bekannten Verfahren, die Gefahr des Verbackens wesentlich geringer ist. Es kann jedes geeignete Trocknungsgerät, das vorzugsweise für Vakuumbetrieb geeignet ist, wie z. B. Schaufeltrockner, Taumeltrockner, Drehrohrofen etc. verwendet werden. Im allgemeinen trocknet man bei Temperaturen um 110 bis 150 °C, bevorzugt 125 bis 140 °C über einen Zeitraum von 3 bis 10 Stunden, bevorzugt 4 bis 8 Stunden unter Anlegen eines geringen Unterdrucks, vorzugsweise 950 bis 980 hPa. Unter diesen Bedingungen wird das gebundene CO₂ wirkungsvoll entfernt. Besonders geeignet ist ein Schaufeltrockner mit randgängigen Schaufeln oder ein Apparat, der die Wärme direkt in das Produkt einbringt.

Das auf diese Weise erhaltene Magnesiumalkoxidpulver kann nach allen gängigen Methoden des Standes der Technik, wie z. B. in der EP-OS 0 236 082 beschrieben, in einen Katalysator für die Polymerisation von α-Olefinen umgesetzt werden. Die damit katalysierte Polymerisation kann auf jede konventionelle Weise durchgeführt werden.

Das erhaltene feinteilige Katalysatorpulver ist besonders geeignet für die Flüssigphasenpolymerisation von α-Olefinen wie z. B. Ethylen, Propylen, Buten-(1), Hexen-(1) oder Mischungen daraus, wobei entweder das verflüssigte Monomer oder ein inerter Kohlenwasserstoff als flüssiges Medium benutzt wird. Die Polymerisation kann hierbei kontinuierlich oder diskontinuierlich durchgeführt werden.

Es ist zu beachten, daß sowohl das hier beschriebene pulverförmige Magnesiumalkoxid als auch der daraus hergestellte Polymerisationskatalysator in verschiedenem Ausmaß empfindlich gegenüber Sauerstoff, Feuchtigkeit, Kohlendioxid, Acetylenen und Schwefelverbindungen sind; daher muß unter sorgfältigem Ausschluß derartiger Katalysatorgifte gearbeitet werden.

Mit derartigen Katalysatoren hergestelltes Polypropylen weist eine hohe Stereospezifität sowie eine hohe Schüttdichte auf und ist bei pneumatischer Förderung leicht fluidisierbar mit einem Lockerungspunkt von weniger als 7 cm/sec, typischerweise weniger als 3 cm/sec und unter geeigneten Bedingungen weniger als 1 cm/sec. Wegen der erfindungsgemäß niedrigen Teilchengröße des Katalysators und der Abwesenheit großer Teilchen besitzt das Polyolefin einen sehr niedrigen Aschegehalt und läßt sich stippenfrei verarbeiten.

### Beispiel 1: Ethanolische Lösung von carboxyliertem Magnesiumethoxid

In einem 3 m³-Rührbehälter werden zu 1 000 kg Ethanol unter Rühren 300 kg Magnesiumethoxid gegeben und anschließend 145 kg CO₂ über ein unter die Flüssigkeitsoberfläche reichendes Tauchrohr so eindosiert, daß keine Gasblasen an der Flüssigkeitsoberfläche erscheinen. Hierbei lösen sich die im Ethanol nahezu unlöslichen Magnesiumethoxidpartikel nach und nach unter Reaktion mit dem CO₂ auf. Es bildet sich eine leicht trübe opaleszierende Lösung von 30,8 Gew.-% carboxyliertem Magnesiumethoxid Mg (OC₂H₅)₂ ^{·} 1,25 CO₂.

### Beispiel 2: Versprühen der Lösung

In einem Sprühturm mit einem Durchmesser- zu Längenverhältnis des zylindrischen Teils von 0,7 werden 12,3 kg/h der Lösung aus Beispiel 1 mittels einer Zweistoffdüse mit Innenzerstäubung gemäß DE-PS 26 27 880 (Last: 7 % der Kapazität) in einen auf 110 °C aufgeheizten Begleitgasstrom (trockenes N₂) versprüht. Die Trockneraustrittstemperatur beträgt dabei ca. 90 °C. Die Partikel werden in einem Doppelzyklon abgeschieden; die Ausbeute beträgt 89,4 %, wobei ohne Feinstaubfilter hinter dem Zyklon gearbeitet wird.

Die entstandenen Partikel aus carboxyliertem Magnesiumethoxid besitzen ein Gewichtsmittel des Teilchendurchmessers von 8,4 µm (bestimmt durch Laserbeugung an einem "Microtrac"-Gerät der Firma Leeds & Northrup). Sie weisen die folgende Korngrößenverteilung auf:
d₁₀ = 3.0 µm; d₅₀ = 6.9 µm; d₉₀ = 15.0 µm ;
d. h. 10 Gew.-% der Teilchen haben einen Durchmesser bis 3 µm usw. Der größte gemessene Durchmesser beträgt 38 µm.

Der Restethanolgehalt beträgt 4.9 Gew.-%, das CO₂ ist noch gebunden.

### Beispiel 3: Trocknung und Decarboxylierung

300 g des Produkts aus Beispiel 2 werden in einem 2 l-Laborschaufeltrockner mit anliegenden PTFE-Schaufeln 5 Stunden bei einem Druck von 980 mbar und einer Innentemperatur von 131 °C im Stickstoffstrom (ca. 10 l/h) nachgetrocknet, wobei Gewichtskonstanz erreicht wird.

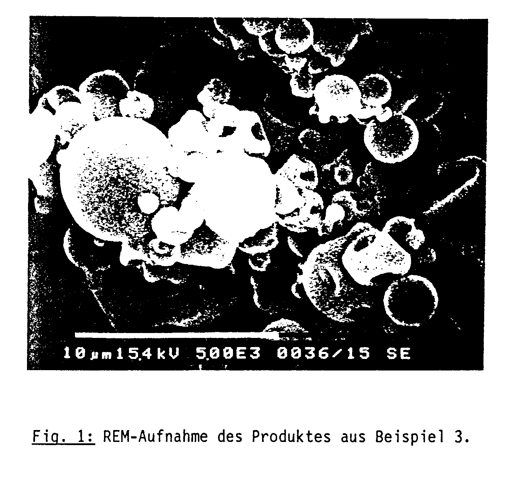

Die Primärkorngrößen verändern sich hierbei nicht; es bilden sich jedoch kleinere Agglomerate, die bei der weiteren Katalysatorpräparation durch Rühren wieder aufgebrochen werden. Durch rasterelektronenmikroskopische Aufnahmen kann nachgewiesen werden, daß die einzelnen kugeligen Partikel durch die Behandlung im Schaufeltrockner nicht zerstört werden (siehe Fig. 1).

Das so erhaltene Magnesiumethoxid wird zur Herstellung eines Katalysators für die Polymerisation von α-Olefinen entsprechend EP-OS 0 236 082 verwendet. Die Aktivität und Stereospezifität des erhaltenen Katalysators sind gut; bei Flüssigphasenpolymerisation von Propylen wird ein Polypropylen von hoher Schüttdichte und guter Fluidisierbarkeit erhalten.

## Patentansprüche

1. Verfahren zur Herstellung von feinteiligem, sphärischem Magnesiumalkoxid Mg(OR)₂ mit R = C₂ bis C₄ Alkyl durch Sprühtrocknen einer Lösung des entsprechenden carboxylierten Magnesiumalkoxids Mg(OR)₂ ^{·} x CO₂ (x = 0,2 bis 2,0) in einem Alkohol ROH und nachfolgende Trocknung und Decarboxylierung,
dadurch gekennzeichnet, daß
a) die Lösung über eine Zweistoffdüse mit Innenzerstäubung, welche im Unterlastbereich mit 5 bis 30 % ihrer Kapazität betrieben wird,
b) in ein inertes Begleitgas versprüht wird, das unter einem Druck von 1,0 bis 1,2 bar steht, auf 100 bis 140 °C vorgeheizt und im Gleichstrom geführt wird,
c) wonach das entstandene feinteilige carboxylierte Magnesiumalkoxid getrocknet und decarboxyliert wird.

2. Verfahren gemäß Anspruch 1,
dadurch gekennzeichnet,
daß die Düse mit 10 bis 25 % ihrer Kapazität betrieben wird.

3. Verfahren gemäß den Ansprüchen 1 und 2,
dadurch gekennzeichnet,
daß als Begleitgas trockener Stickstoff verwendet wird.

4. Verfahren gemäß den Ansprüchen 1 bis 3,
dadurch gekennzeichnet,
daß der Sprühtrockner aus einem oberen zylindrischen Teil mit einem Durchmesser- zu Längenverhältnis von 0,3 bis 0,9, vorzugsweise 0,6 bis 0,8, sowie einem anschließenden konischen Teil besteht.

5. Verfahren gemäß Anspruch 4,
dadurch gekennzeichnet,
daß der konische Teil des Sprühtrockners länger als der zylindrische ist.

6. Verfahren gemäß den Ansprüchen 1 bis 5,
dadurch gekennzeichnet,
daß R = Ethyl ist.

7. Verfahren gemäß Anspruch 6,
dadurch gekennzeichnet,
daß x = 1,0 bis 1,25 ist.

8. Verfahren gemäß den Ansprüchen 1 bis 7,
dadurch gekennzeichnet,
daß das Begleitgas auf 105 bis 120 °C vorgeheizt wird.

9. Gemäß den Ansprüchen 1 bis 8 hergestelltes feinteiliges, sphärisches Magnesiumalkoxid,
dadurch gekennzeichnet,
daß das Gewichtsmittel des Teilchendurchmessers weniger als 10 µm beträgt.

10. Verwendung des Magnesiumalkoxids gemäß Anspruch 9 zur Herstellung eines Katalysators für die Polyerisation von α-Olefinen.

## Claims

1. A process for the preparation of a finely divided, spherical magnesium alkoxide Mg(OR)₂ in which R is C₂- to C₄-alkyl by spray-drying of a solution of the corresponding carboxylated magnesium alkoxide Mg(OR)₂ · x CO₂ (x = 0.2 to 2.0) in an alcohol ROH and subsequent drying and decarboxylation,
characterised in that
a) the solution is sprayed via a two-material nozzle with inner atomisation, which is operated in the underload region at 5 to 30% of its capacity,
b) into an inert accompanying gas which is at a pressure of 1.0 to 1.2 bar, is preheated to 100 to 140°C and is fed cocurrent,
c) after which the finely divided carboxylated magnesium alkoxide formed is dried and decarboxylated.

2. A process according to claim 1,
characterised in that the nozzle is operated at 10 to 25% of its capacity.

3. A process according to either of claims 1 and 2, characterised in that the accompanying gas used is dry nitrogen.

4. A process according to any of claims 1 to 3, characterised in that the spray dryer is composed of an upper cylindrical part having a ratio of diameter to length of 0.3 to 0.9, preferably 0.6 to 0.8, and a subsequent conical part.

5. A process according to claim 4, characterised in that the conical part of the spray dryer is longer than the cylindrical part.

6. A process according to any of claims 1 to 5, characterised in that R is ethyl.

7. A process according to claim 6, characterised in that x is 1.0 to 1.25.

8. A process according to any of claims 1 to 7, characterised in that the accompanying gas is preheated to 105 to 120°C.

9. A finely divided, spherical magnesium alkoxide prepared according to any of claims 1 to 8, characterised in that the weight average of the particle diameter is less than 10 µm.

10. Use of the magnesium alkoxide according to claim 9 for the preparation of a catalyst for the polymerisation of α-olefins.

## Revendications

1. Procédé de préparation d'alcoxyde de magnésium sphérique, finement divisé, de formule
Mg(OR)₂,
dans laquelle R représente un radical alkyle comportant de 2 à 4 atomes de carbone, en séchant par pulvérisation une solution (dans un alcool ROH) de l'alcoxyde de magnésium carboxylé correspondant Mg(OR)₂ . x CO₂ (x ayant une valeur de 0,2 à 2,0), puis en séchant ensuite et en décarboxylant, caractérisé par le fait :
a) que la solution est projetée, par l'intermédiaire d'une buse à deux substances à pulvérisation interne qui, dans la zone de charge inférieure, fonctionne à 5 - 30 % de sa capacité,
b) dans un gaz d'accompagnement inerte se trouvant sous une pression de 1,0 à 1,2 bar, préchauffé à une température de 100 à 140°C et guidé à courant de même sens,
c) et que l'alcoxyde de magnésium carboxylé, qui s'est formé à l'état finement divisé, est ensuite séché et décarboxylé.

2. Procédé selon la revendication 1,
caractérisé par le fait que la buse fonctionne à 10 - 25 % de sa capacité.

3. Procédé selon les revendications 1 et 2,
caractérisé par le fait que l'on utilise de l'azote sec comme gaz d'accompagnement.

4. Procédé selon les revendications 1 à 3,
caractérisé par le fait que le dispositif de séchage par pulvérisation est constitué par une partie supérieure cylindrique d'un diamètre faisant de 0,3 à 0,9, de préférence de 0,6 à 0,8 fois sa longueur, ainsi que par une partie conique s'y raccordant.

5. Procédé selon la revendication 4,
caractérisé par le fait que la partie conique du dispositif de séchage par pulvérisation est plus longue que la partie cylindrique.

6. Procédé selon les revendications 1 à 5,
caractérisé par le fait que R représente un radical éthyle.

7. Procédé selon la revendication 6,
caractérisé par le fait que x a une valeur de 1,0 à 1,25.

8. Procédé selon les revendications 1 à 7,
caractérisé par le fait que le gaz d'accompagnement est préchauffé à une température de 105 à 120°C.

9. Alcoxyde de magnésium sphérique, finement divisé, préparé selon les revendications 1 à 8,
caractérisé par le fait que le diamètre moyen en masse des particules est inférieure à 10 µm.

10. L'utilisation de l'alcoxyde de magnésium selon la revendication 9 pour préparer un catalyseur pour la polymérisation d'alpha-oléfines.
